# EUROPEAN PATENT SPECIFICATION

(11) **EP 2 361 654 B1**
(45) Date of publication and mention of the grant of the patent: **03.06.2015**
(21) Application number: 10153978.1
(22) Date of filing: 18.02.2010
(51) Int. Cl.: A61N 1/372

(54) **Wakeup of implantable communication circuitry**
Réveil de circuit de communication implantable
Aufwecken einer implantierbaren Kommunikationsschaltung

(43) Date of publication of application: 31.08.2011
(73) Proprietor: St. Jude Medical AB, 175 84 Järfälla (SE)
(72) Inventor: Wikman, Birgitte, 187 43 Täby (SE); Franzèn, Johan, 413 11 Göteborg (SE)
(74) Representative: Sjölander, Henrik

(56) References cited:
- WO-A1-2006/116004
- WO-A1-2008/118045
- US-A1- 2003 119 568
- US-A1- 2008 021 972
- US-A1- 2008 215 121

## Description

### TECHNICAL FIELD

The present invention generally relates to communication with implantable medical devices, and in particular to waking up the communication circuitry of implantable medical devices to enable wireless communication.

### BACKGROUND

The traditional approach of conducting communication with an implantable medical device (IMD) has been through usage of inductive telemetry. Currently, the inductive telemetry is replaced by radio frequency (RF) based communication protocols. Usage of RF carriers provides longer communication distances but also drains more energy from the battery-powered IMD, which thereby has negative impact on the longevity and service life of the IMD.

As a consequence, the communication circuitry of an IMD is typically kept in an inactive, low power state between communication sessions. Today, different approaches have been suggested to wake up the IMD circuitry from the inactive state to an active state. An approach has been to schedule communication sessions to pre-defined communication time periods. This, however, requires synchronized clocks in the IMD and a non-implantable communication device to thereby become active at the same time. The high-precision clock in the IMD has to be regularly synchronized to be useful. A clock synchronization mechanism is, though, complicated and costly in terms of resource utilization for the battery-powered IMD having limited processing resources, memory and power supply. A further limitation of this approach is that it is not possible to set up a communication session on the demand but merely at the scheduled time periods.

An alternative approach has therefore been to enable the IMD to receive dedicated wakeup messages even when the communication circuitry is in the inactive state. Upon reception of such a wakeup message from the communication device, the IMD reactivates its communication circuitry to thereby be able to wirelessly communicate with the communication device.

Traditionally, the wakeup messages are sent from the communication device on a regular basis and with a low periodicity, such as about once a day, in order to collect information from the IMD. Wakeup messages can also be sent on demand, for instance in connection with visits at the patient's physician.

It may, however, sometimes be necessary for the IMD to upload information to the communication device with short notice, such as following detection of a critical heart condition. Today there are communication circuitries on the market that allow the IMD itself to switch from the inactive state to the active state. The IMD can then send data packets with the desired information to the communication device. However, it could be possible that the IMD is not within communication range to the communication device when it itself activates its communication circuitry. In such a case, the IMD will try to retransmit data packets several times before concluding that no communication session can be established. This emergency approach therefore will consume at lot of battery power in vain in most of the cases when the patient is not present at home or at a health care facility where a communication device is present.

US 2009/0248115 discloses an IMD with a telemetry module with a configurable polling interval at which the telemetry module is powered up from an inactive state to perform sniff operations for detecting whether communication signals are received from a communication device. The polling interval of the telemetry module is configured based on a parameter sensed by a sensor of the IMD, such as a motion sensor, a position sensor, a patient activity sensor or a magnetic sensor.

US 7,359,753 discloses a communication device transmitting a repeating sequence of special wakeup characters in order to establish a communication session with an IMD. The communication device can be programmed to adjust the periodic interval at which it transmits the repeating sequence to the IMD in accordance with when previous successful communication sessions have been established.

US 2008/0215121 discloses enabling RF communication between an IMD and an external device by duty cycling the RF circuitry of the IMD. The external device transmits a data segment containing a repeating sequence of special wakeup characters in order to establish a communication session with the IMD.

US 2003/0119568 discloses a wireless communications apparatus with a wakeup receiver adapted to receive a wakeup signal and an initially powered-off transceiver. When the wakeup receiver receives the wakeup signal it can in turn power on the transceiver.

Embodiments as disclosed herein solve the problems of the prior art techniques. The embodiments in particular reduce the risk of draining the IMD battery in connection with IMD-initiated activation of communication circuitry and communication session establishment.

It is an objective to provide an efficient wakeup of IMD communication circuitry.

It is a particular objective to provide a technique for establishing a communication session with an IMD with reduced power consumption by the IMD.

These and other objectives are met by embodiments as disclosed in the present description.

Briefly, a communication device is configured to conduct wireless communication with an IMD. The communication device comprises a transmitter and a receiver connected to an antenna. A wakeup processor is provided to generate wakeup messages. These wakeup messages are of at least two types. First wakeup messages are generated by the wakeup processor to comprise a first identifier code associated with the IMD. Corresponding second wakeup messages from the wakeup processor comprise a second, different identifier code associated with the IMD. The transmitter is then configured to transmit the first and second wakeup messages to the IMD.

The IMD comprises a communication circuitry, with a transmitter, configured to conduct wireless communication with the communication device. A wakeup controller controls the operation state of the communication circuitry to switch the state between an inactive, low power state and an active state based on reception of a wakeup message comprising an identifier code currently assigned to the IMD. A code processor of the IMD is configured to select one of a first and second identifier code from a memory as the identifier code currently assigned to the IMD.

Thus, depending on the actual choice of identifier code of the IMD, the IMD responds to first wakeup messages or the second wakeup messages and activates its communication circuitry based on a wakeup message with the correct identifier code.

The usage of multiple identifier codes for an IMD enables the IMD to control when it wants to initiate a communication session with the communication device, by either responding to the first wakeup messages or the second wakeup messages. In a particular embodiment, the transmitter of the communication device transmits the second wakeup messages more frequently than the first wakeup messages. The IMD can therefore select the second identifier code as the currently assigned identifier code if it wants to quickly establish a communication session with the communication device.

The energy consumption in connection with trying to set up a communication session is thereby significantly reduced as compared to the prior art situation where the IMD may transmit a lot of session requests in vain if no communication device is present within communication session. According to the present solution, no such unnecessary transmissions are required as the communication session will be established very shortly after the IMD comes into communication range with the communication device by being able to listen and respond to the more frequently transmitted wakeup message type.

An aspect of the invention relates to a method of enabling wakeup of an IMD to conduct wireless communication with the IMD. The method involves generating first wakeup messages comprising the first identifier code and second wakeup messages comprising the second identifier code associated with the IMD. The first wakeup messages and the second wakeup messages are then transmitted, preferably with different periodicities.

A further aspect of the invention relates to a method of waking up an IMD and involves selecting one of a first and second identifier code as the identifier code currently assigned to the IMD. A wakeup message comprising an identifier code is received and compared to the identifier code currently assigned to the IMD. If the two codes are equal, the communication circuitry of the IMD is switched from an inactive state to an active state to enable establishment of a communication session.

### BRIEF DESCRIPTION OF THE DRAWINGS

The invention, together with further objects and advantages thereof, may best be understood by making reference to the following description taken together with the accompanying drawings, in which:
Fig. 1 is an overview of the communication between an implantable medical device and a non-implantable communication device;
Fig. 2 is a schematic block diagram of an embodiment of a communication device;
Fig. 3 is a schematic block diagram of an embodiment of an implantable medical device;
Figs. 4A-4C illustrate signaling of wakeup messages and wakeup of the communication circuitry of an implantable medical device according to different embodiments;
Fig. 5 is a flow diagram illustrating a method of enabling wakeup of the communication circuitry of an implantable medical device;
Fig. 6 is a flow diagram illustrating additional, optional steps of the method illustrated in Fig. 5;
Fig. 7 is a flow diagram illustrating a method of waking up the communication circuitry of an implantable medical device;
Fig. 8 is a flow diagram illustrating additional steps of the method in Fig. 7; and
Fig. 9 is a flow diagram illustrating additional steps of the method in Fig. 8.

### DETAILED DESCRIPTION

Throughout the drawings, the same reference numbers are used for similar or corresponding elements.

The present embodiments generally relate to communication between an implantable medical device (IMD) and an external, non-implantable communication device. In particular, embodiments disclose a technique allowing efficient wakeup of an IMD from an inactive state in which communication circuitry of the IMD is kept in an inactive or low power state to an active state in order to enable wireless radio frequency (RF) based communication between the IMD and the communication device.

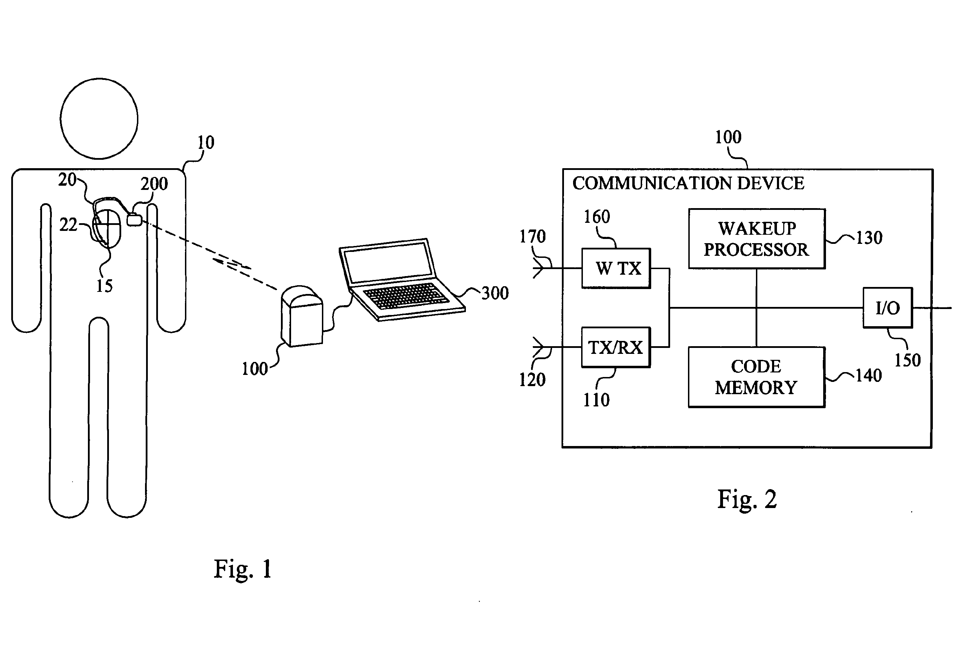

Fig. 1 is a schematic overview of a data communication system according to an embodiment. The data communication system comprises an IMD 200, illustrated as being implanted in a human body 10 in the figure. The IMD 200 of the embodiments can actually be any implantable medical device capable of delivering therapy to an animal, preferably mammalian and more preferably human body 10, and/or capable of recording physiological data and parameters from the body 10. The figure non-limitedly illustrates the IMD 200 as a device monitoring and/or providing therapy to the patient's heart 15 and consequently comprises one or more connectable cardiac leads 22 provided in or in connection to one or more ventricles and/or atriums of the heart 15. The IMD 200 could therefore be a pacemaker, defibrillator or cardioverter. However, the present invention is not limited to cardiac-associated IMDs 200 but may also be practiced with other implantable medical devices 200, such as drug pumps, neurological stimulators, physical signal recorders, oxygen sensors, or the like. The important feature of the IMD 200 is that it contains equipment capable of conducting wireless RF-based communication with the communication device 100 of the data communication system.

The communication device 100 operates as a base station of the data communication system in that it constitutes the interface between the IMD 200 and an external instrument or data processing unit 300, such as a programmer for the IMD 200. This means that the communication device 100 contains the equipment for effecting the wireless RF-based communication with the IMD 200 on behalf of the data processing unit 300. Thus, data requests from the data processing unit 300 are processed and packed into data packets and transmitted to the IMD 200 by the communication device 100. Additionally, data packets received from the IMD 200 by the communication device 100 can be forwarded to the data processing unit 300 for further processing and/or display therein.

The communication device 100 and the data processing unit 300 can be separate devices as illustrated in Fig. 1, either wired connected or using a wireless connection, such as Bluetooth^{®}, an infrared (IR) connection or an RF connection. In an alternative embodiment, the functionality and equipment of the communication device 100 and the data processing part of the data processing unit 300, can be housed in a same device, such as a physician's programmer or workstation. The programmer can additionally comprise or be connected to a display screen for displaying the physiological data collected by the IMD 200 and wirelessly transmitted to the communication device 100 and processed by the data processor of the programmer. The communication device 100 and data processing unit 300 could also form a single, portable unit that can be carried by the patient, if desired.

RF-based communication with an IMD introduces new challenges as compared to inductive telemetry. Generally, RF telemetry requires more power than inductive telemetry, which drains the battery driven IMD and has a negative impact on the longevity and service life of the IMD. Consequently, power-saving algorithms have been developed. An example of such a power saving algorithm is to keep the communication circuitry of the IMD in a low power or inactive state or mode between communication sessions. In the inactive state only a dedicated low power wakeup receiver is, at least temporarily, active in order to sniff for and capture wakeup messages transmitted from the communication device. Thus, when there is no communication session present between the IMD and the communication device, the communication circuitry of the IMD is kept completely or partly turned off in order to save power of the IMD battery.

Embodiments as disclosed herein involve a new and improved technique of effecting the wakeup of the IMD communication circuitry to start a communication session involving transmission of data between the IMD and the communication device and further up to or from the data processing unit.

In the prior art, each IMD has a single assigned identifier code that it responds to. Thus, only wakeup messages comprising this particular identifier code triggers a response in the IMD, i.e. wake up of the communication circuitry. The embodiments have taken a radically different approach by assigning multiple, i.e. at least two such identifier codes, for a single IMD. The communication device can thereby generate and transmit two types of wakeup messages; one type comprises a first identifier code and the other two comprises a second identifier code. The IMD can in turn affect its wakeup pattern by switching between these at least two identifier codes as the identifier code currently assigned to the IMD.

For instance, the communication device could transmit the first wakeup messages with the first identifier code with a first periodicity or frequency, i.e. time interval between transmission occasions, and transmit the second wakeup messages with the second identifier code with a second, different periodicity. If the IMD has a need to quickly send data to the communication device for further forwarding to the data processing unit, the IMD selects the second identifier code as the identifier code currently assigned to the IMD if the second wakeup messages are sent more frequent than the first type. When the IMD does not have any imminent need for uploading data to the data processing unit through the communication device it instead selects the first identifier code as the currently assigned identifier code.

Even if the IMD has access to multiple identifier codes, it preferably only uses one of them at a time as its currently assigned identifier code. This means that the IMD will only wake up its communication circuitry in response to a correct reception of a wakeup message comprising the identifier code that is currently assigned to the IMD. A wakeup message comprising the other identifier code currently not assigned to the IMD will therefore be ignored and does not trigger activation of the IMD communication circuitry.

Fig. 2 is a schematic block diagram of an embodiment of the communication device 100 capable of conducting wireless RF-based communication with an IMD. The communication device 100 in particular comprises communication circuitry 110, 120, 160, 170 for enabling communication with the IMD. In an embodiment, the communication device 100 comprises a dedicated wakeup transmitter 160 connected to an RF antenna 170. This wakeup transmitter 160 is then configured to only transmit the so-called wakeup messages in order to active the IMD communication circuitry and establish a communication session. Once successful activation of the IMD communication circuitry has been triggered, another transmitter 110 and a receiver 110 connected to an RF antenna 120 are utilized to transmit and receive data packets to and from the IMD, respectively. In the figure the transmitter and receiver have been illustrated as the transmitting branch and the receiving branch of a combined RF transmitting and receiving unit or transceiver 110. Alternatively, the transmitter and receiver can be implemented as separate units connected to separate RF antennas or to a common RF antenna.

Usage of a dedicated wakeup transmitter 160 is generally advantageous since then different parts of the frequency spectrum can be utilized for wakeup messages as compared to the communication of data packets during a communication session. As a non-limiting example, the wakeup transmitter 160 could use the 2.45 GHz Industrial, Scientific and Medical (ISM) radio band (frequency range of 2.4-2.5 GHz and center frequency of 2.45 GHz). The transmitter 110 and receiver 110 could then utilize the frequency interval from 400 to 410 MHz, preferably 402-405 MHz capable of serving ten Medical Implant Communication Service (MICS) channels, or 433.05 to 434.79 MHz (center frequency 433.92 MHz) capable of serving two ISM channels. It is though anticipated by the embodiments that other frequency bands than MICS and ISM can be used by the communication device 100 and the IMD.

In an alternative embodiment, the communication device 100 comprises the receiver 110 and a transmitter 110 connected to separate RF antennas or a common RF antenna 120 or a transceiver 110 with its connected RF antenna 120. This embodiment consequently does not have any dedicated wakeup transmitter that is employed for transmission of the wakeup messages. In clear contrast, the transmitter 110 then operates for both transmitting the wakeup message and sending data packets during an established communication session. The transmitter 110 could use a same frequency interval or intervals for both the wakeup messages and the other communication or is configured to transmit with a carrier frequency in different frequency intervals depending on whether wakeup messages or other data packets are transmitted.

In these alternative embodiments, the receiver 110 and transmitter(s) 110, 160 are preferably all implemented on a communication or transceiver chip. Generally, the communication chip, e.g. MICS cip, comprises the functionalities for defining the frequency band that is used for the RF-based communication, establishing the radio link, etc. MICS chips are currently available from vendors such as Zarlink Semiconductor, such as Zarlink 701 xx chips.

The communication device 100 comprises a wakeup processor 130 that can also be implemented on the communication chip. The wakeup processor 130 is configured to generate the wakeup messages. According to the embodiments, the wakeup processor 130 generates at least wakeup messages of a first type, denoted first wakeup messages herein, and wakeup messages of a second type, so called second wakeup messages. The first wakeup messages comprises a first identifier code associated with an IMD and the second wakeup messages comprises a second, different identifier code associated with the IMD.

The respective identifier codes are preferably stored in a code memory 140 accessible for the wakeup processor 130 and which advantageously is implemented on the communication chip. The communication device 100 could be configured to only be able to communicate with a single IMD. In such a case the code memory 140 comprises the at least two identifier codes that this IMD can potentially respond to. If the communication device 100 instead is potentially able to communicate with multiple IMDs, the code memory 140 preferably stores respective identifier codes together with respective device identifiers of the different IMDs. Thus, the code identifiers associated with an IMD are further associated in the code memory 140 with device identifier of the IMD. This enables the wakeup processor 130 to retrieve correct identifier code from the code memory 140 based on a given device identifier. In an alternative approach, the code identifiers are used or comprise device identifiers so no separate such device identifiers need to be stored in the code memory 140.

The wakeup transmitter 160, or alternatively the transmitter 110 if no dedicated wakeup transmitter 160 is available, is configured to transmit the first wakeup messages comprising the first identifier code and the second wakeup messages comprising the second identifier code to the IMD in order to enable wakeup of the IMD communication circuitry. In the following description it is assumed that the communication device 100 uses a dedicated wakeup transmitter 160 for transmission of wakeup messages whereas another transmitter 110 performs the transmission of data or radio packets in a communication session. This should however merely be seen as an illustrative but non-limiting implementation embodiment.

The transmission of the at least two types of wakeup messages can be performed according to various embodiments. In a particular embodiment or default mode, the wakeup transmitter 160 is configured to transmit the first wakeup messages with a first periodicity and transmit the second wakeup messages with a second, different periodicity. The first wakeup messages could be used as default wakeup messages that are sent fairly seldom in order to regularly interrogate the IMD for the purpose of collecting diagnostic and/or device data registered by the IMD and/or sending programming commands to the IMD. Such regular communication is typically conducted once or twice a day. For instance, the communication device 100 can form part of a home monitoring unit that is designed to collect diagnostic data recorded by the IMD during the day. The diagnostic data can then be stored in the home monitoring unit or is forwarded to a computer or server of a healthcare facility for usage by the patient's physician. In such a case, IMD interrogations are typically conducted in the patient's home during night time, when it is likely that the patient and the IMD is present within communication distance from the home monitoring unit. The wakeup processor 130 generates a first wakeup message comprising the first or default identifier code associated with the IMD and the wakeup transmitter 160 transmits this first wakeup message at one or more time occasions during the night. If the IMD is present at home and has selected the first identifier code as the identifier code currently assigned to the IMD, it will capture the wakeup message and activate its communication circuitry.

The second wakeup messages could then be regarded as complementary or emergency wakeup messages that are preferably transmitted more often than the first wakeup messages. For instance, these second wakeup messages could be transmitted from once per second up to about once per hour, with a typical frequency of once per 30 s up to once per 5 minutes.

The IMD can use this fact when it needs to quickly upload data to the communication device 100 and further to the data processing unit. For instance, the IMD might have detected a critical medical condition of the patient or some serious malfunction of the IMD, such as low battery power. In such a case, it could be very important that this information is quickly communicated to the data processing unit in order to alert the patient and/or the physician. The relative long time periods between transmission occasions for the first wakeup messages, such as 12-24 hours, could be too long in the present case. The IMD instead assigns the second identifier code as its currently assigned identifier code to thereby active its communication circuitry based on the more frequent second wakeup messages.

In the art, the IMD could by itself activate its communication circuitry based on an emergency event. However, this could lead to a situation where the communication circuitry is activated even when there is no communication device 100 present within communication distance. In such a case, the IMD tries to establish a communication session by transmitting and retransmitting session requests to the communication device 100. This, however, consumes power from the IMD battery and this power drain will be in vain when the IMD is not close to the communication device 100.

If the communication device 100 instead transmits the second wakeup messages more frequently than the default wakeup messages (first type), the IMD can select to change from the first identifier code to the second identifier code when it wants to establish a communication session with the communication device 100, such as following an emergency event. If the communication device 100 is within communication distance, the IMD will shortly receive a second wakeup message comprising the second identifier code that it has selected. The IMD then activates its communication circuitry. If there is no communication device 100 within communication distance, the IMD will not receive any second (or indeed first) wakeup message but will not waste any valuable battery power by trying to establish a communication session with a communication device 100 that is not present.

Thus, in an implementation embodiment of the multiple types of wakeup messages, one of the wakeup message types can be utilized as a default wakeup message transmitted regularly or intermittent but with a rather low frequency, i.e. long time period between transmission occasions. The second wakeup messages are then utilized as complement or emergency wakeup messages that are preferably transmitted more frequently and can be used by the IMD to quickly establish a communication session.

Another usage of the multiple wakeup messages types is to quickly establish a communication session with the IMD. This can be done since the IMD will have one of the at least two identifier codes used as the currently assigned identifier code. The communication device 100, however, does not know which of the identifier codes that the IMD currently uses. In such a case, the wakeup transmitter 160 can alternate between transmitting a first wakeup message with the first identifier code and a second wakeup message with the second identifier code. The IMD responds to one of these types of wakeup messages carrying the identifier code that is identical to the currently assigned identifier code of the IMD. Thus, as soon as the IMD correctly receives a wakeup message with a relevant identifier code it will active its communication circuitry and can start to communicate with the communication device 100.

This alternative implementation embodiment can be used together with the periodic transmission of the first and second wakeup messages. In such a case, the transmission of the first and second wakeup messages at different periodicities could be utilized by the communication device 100 as a default mode. However, when the communication device 100 receives data from the connected data processing unit that needs to be downloaded to the IMD it can enter a communication mode. In this communication mode, the wakeup transmitter 160 temporarily stops the periodic transmission of the first and second wakeup messages and preferably alternate between transmitting the first and second wakeup messages. The same high frequency used in the default mode for the second wakeup messages could also be used in the communication mode for the first and second wakeup messages, such as preferably from once per 30 s up to once per 5 minutes. Alternatively, the wakeup messages are transmitted even more frequently in the communication mode.

Once the communication device 100 has established a communication session with the IMD and downloaded the data from the data processing unit, it advantageously leaves the communication mode and the alternate transmission of the first and second wakeup messages to thereby once more enter the default mode. The communication device 100 continues with the periodic transmission of the first and second wakeup messages, where the periodicity of the two types of wakeup messages are preferably different.

In a particular embodiment, the wakeup processor 130 or some other unit (not illustrated in Fig. 2) of the communication device 100 is advantageously configured to generate a code setting command in connection with or following the end of a communication session with the IMD. The transmitter 110 transmits this code setting command to the IMD. The code setting command triggering selection, at the IMD, of the first identifier code as the identifier code currently assigned to the IMD. Thus, if the IMD prior to the established communication session had the second identifier code as its currently assigned identifier code, the code setting command triggers a switch to the first identifier code. If the IMD instead already used the first identifier code at the start of the communication session, it can simply ignore the code setting command.

The reason behind usage of the code setting command is that it triggers the IMD to use the default identifier code following the established communication session. This is advantageous if the second identifier code and the second wakeup messages are to mainly be used in connection with emergency events, when the IMD quickly needs to establish a communication session with the communication device 100. Once such a communication session has been ended the emergency mode should also be ended, through the potential change of currently assigned identifier code to the first or default identifier code. If a second emergency event occurs shortly, the IMD can therefore change back to the second identifier code to thereby quickly initiate a new communication session with the communication device 100. If no change in identifier codes would have occurred, a communication session could have been established very shortly after the first communication session ended. However, at that point the IMD probably has no need for communicating with the communication device 100 as the second emergency event has not yet occurred. Thus, in a preferred implementation of this mode, the second identifier code is mainly used by the IMD when it urgently needs to communicate with the communication device 100 and should therefore preferably not be used anymore after a communication session has ended.

The communication device 100 also comprises a general input and output (I/O) unit 150 operating as the interface between the communication device 100 and the connected data processing unit. The I/O unit 150 can, in particular in the case of a wired connection, represent the equipment of the communication device 100 allowing forwarding of data from the communication device 100 to the data processing unit and vice versa over the wired connection. In the case of a wireless communication, the I/O unit 150 represents the equipment, such as transmitter/receiver and antenna, required in order to effectuate such a wireless data transfer.

The units 110, 130, 140, 160 of the communication device 100 are preferably implemented in hardware on a communication chip. The I/O unit 150 may also be implemented in hardware on the same chip or in a separate hardware structure of the communication device 100. Functionalities of the wakeup processor 130, the transmitters 110, 160 and the receiver 110 may alternatively be at least partly implemented in software.

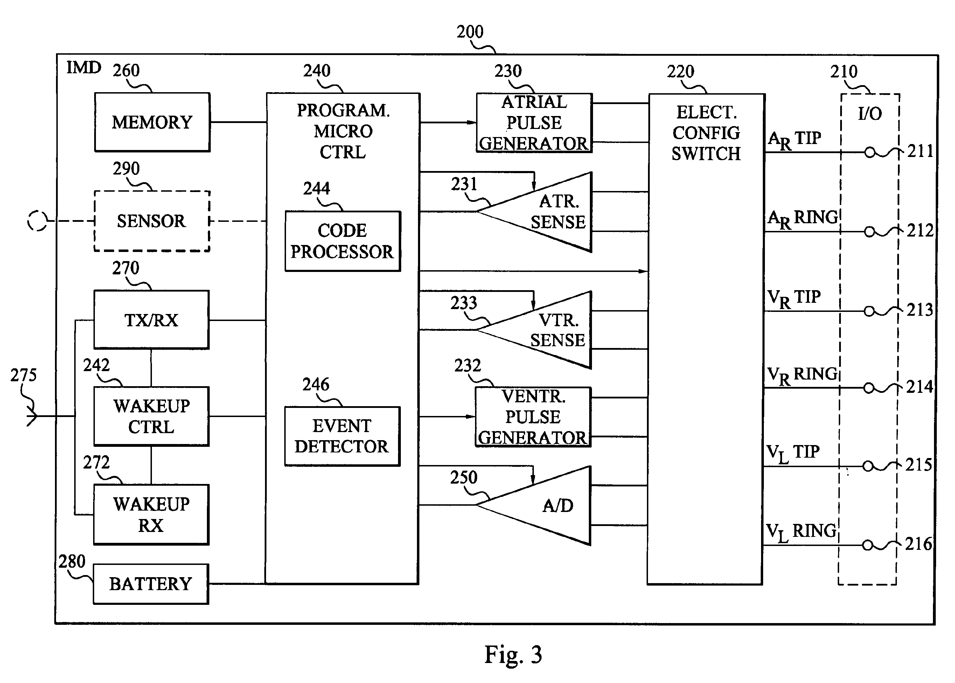

Fig. 3 illustrates an embodiment of an IMD 200, exemplified by a device suitable for delivering cardiac therapy to a heart of a subject. The IMD 200 in particular comprises a transmitter 270 connected to an RF antenna 275 and a receiver 270 connected to an RF antenna 275. The transmitter and receiver 270 are employed by the IMD 200 for transmission and reception of data from the communication device during an established communication session. The transmitter and receiver 270 can be implemented as separate units or can represent the transmitting and receiving branches of a common transceiver 270 as illustrated in the figure. If provided as separate units, they may comprise separate RF antennas or share a common RF antenna 275.

In a preferred embodiment, the IMD 200 additionally comprises a dedicated wakeup receiver 272 that is configured to receive wakeup messages from the communication device. This means that the wakeup receiver 272 is at least partly active even during the inactive, low-power state, during which the transceiver 270 or at least a part thereof is preferably kept inactive to save power of a battery 280 of the IMD 200.

The dedicated wakeup receiver 272 can have a separate RF antenna or is connected to the (common) RF antenna 275 of the transceiver 270.

In an alternative embodiment, the IMD 200 does not have a separate wakeup receiver 272. The separate receiver or the receiving branch of the transceiver 270 is then employed to be at least partly active during the inactive state in order to be able to capture wakeup messages. In such a case, remaining portions of the transceiver 270 not involved in the reception of wakeup messages are preferably kept inactivated or at low power during the inactive state.

It is though generally preferred to have a separate wakeup transceiver 272 as illustrated in the figure since then the complete communication circuitry utilized for data communication with the communication device can be kept inactivated to thereby only have the wakeup receiver 272 draining a very low amount of power of the battery active to capture wakeup messages. In the following discussion it is assumed that the IMD 200 comprises a dedicated wakeup receiver 272 as illustrated in the figure.

The transceiver 270 could operate at one frequency interval, such as the previously mentioned 400-410 MHz, preferably 402-405 MHz, or 433.05-434.79 MHz interval. The wakeup receiver 272 could then utilize a different frequency (interval), such as 2.4-2.5 GHz.

The wakeup receiver 272 and the transceiver 270 are connected to a wakeup controller 242 that is configured to control the operation of the transceiver 270 and in particular change the operation state of the transceiver 270 between inactive and active states based on reception by the wakeup receiver 272 of wakeup messages. Thus, if the wakeup receiver 272 receives a wakeup message comprising an identifier code that is identical to the identifier code currently assigned to the IMD 200, the wakeup controller 242 (re)activates the transceiver 270 so that is in an active state to be able to wirelessly communicate with the communication device. Correspondingly, following the end of a communication session with the communication device, the wakeup controller 242 preferably controls the transceiver 270 to once more enter the inactive, low power state.

In a preferred embodiment, the wakeup controller 242 controls the operation state of the transceiver 270, i.e. both the transmitting and receiving branches. In an alternative approach that does not save as much power during the inactive state, the wakeup controller 242 only affects the operation state of the transmitter/transmitting branch or a portion thereof or the receiver/receiving branch or a portion thereof. However, it is generally preferred, in order to save as much power as possible in the inactive state, to temporarily power down the complete transceiver 270 instead of merely a portion thereof.

The IMD 200 also comprises a memory 260 for storing at least a first identifier code and a second identifier code that can be used by the IMD 200 as the currently assigned identifier code. A code processor 244 is implemented in the IMD, such as a part of a controller 240. The code processor 244 is configured to access the memory 260 and select one of the first and second identifier codes as the identifier code currently assigned to the IMD 200. The identifier code selected by the code processor 244 from the ones stored in the memory 260 affects the operation of the wakeup receiver 272 and the wakeup controller 242. Thus, the currently assigned identifier code determines whether a received wakeup message is recognized or should be ignored. Thus, if the wakeup message comprises an identifier code that is identical to the identifier code selected by the code processor 244 as the currently assigned identifier code, the wakeup controller 242 controls the transceiver 270 to switch from the inactive state to the active state. If the identifier code of the wakeup message is not identical to the currently assigned identifier code, the wakeup receiver 272 ignores the wakeup message and does not trigger the wakeup controller 242 to perform any transceiver activation.

The code processor 244 is preferably configured to select the first identifier code as the default identifier code of the IMD 200. The second identifier code can then be utilized, for instance, in connection with emergency events as is further described herein.

The code processor 244 is preferably responsive to code setting commands received by the transceiver 270 from the communication device. Such a code setting command instructs the code processor 244 which identifier code to currently use for the IMD 200. In preferred implementations, the code setting commands triggers the code processor 244 to select the first or default identifier code. This is in particular advantageously following a previous emergency period in which the code processor 244 has selected the second identifier code to thereby quickly initiate a communication session between the transceiver 270 and the communication device. Once the data packets relating to the emergency event has been sent to the communication device by the transceiver 270, a received code setting command triggers a switch back to the default identifier code to enable the code processor 244 the possibility of once more switch to the second identifier code in response to detection of a new emergency event.

Fig. 3 additionally depicts various other components of the IMD 200. While a particular multi-chamber device is shown in the figure, it is to be appreciated and understood that this is done merely for illustrative purposes. Thus, the techniques and methods described herein can be implemented in connection with other suitably configured IMDs. Accordingly, the person skilled in the art can readily duplicate, eliminate, or disable the appropriate circuitry in any desired combination to provide an IMD capable of treating the appropriate heart chamber(s) with pacing stimulation and optionally also cardioversion and/or defibrillation. The IMD 200 must further not necessarily be a pacemaker or other cardiac stimulating device. In clear contrast, any implantable medical device comprising equipment for enabling RF-based, wireless communication with the communication device is encompassed by the definition of IMD 200 as used herein.

The IMD 200 comprises a housing, often denoted as can or case in the art. The housing can act as return electrode for unipolar leads, which is well known in the art. The IMD 200 also comprises a lead connector or input/output (I/O) 210 having, in this embodiment, a plurality of terminals 211-216. The lead connector 210 is configured to be, during operation in the subject body, electrically connectable to at least one cardiac lead, such as at least one atrial lead, a right ventricular lead and a left ventricular lead. The lead connector 210 consequently comprises terminals 211, 212 that are electrically connected to matching electrode terminals of an atrial lead when the atrial lead is introduced in the lead connector 210. For instance, one of these terminals 211 can be designed to be connected to a right atrial tip terminal of the atrial lead, which in turn is electrically connected through a conductor running along the lead body to a tip electrode present at the distal end of the atrial lead in the right atrium of the heart. A corresponding terminal 212 is then connected to a right atrial ring terminal of the atrial lead that is electrically connected by another conductor in the lead body to a ring electrode present in connection with the distal part of the atrial lead, though generally distanced somewhat towards the proximal lead end as compared to the tip electrode.

In an alternative implementation, the IMD 200 is not connectable to a right atrial lead but instead to a left atrial lead configured for implantation in the left atrium. A further possibility is to have an IMD 200 with a lead connector 210 having sufficient terminals to allow the IMD 200 to be electrically connectable to both a right atrial lead and a left atrial lead. It is also possible to have a lead connector 210 without any terminals for any atrial leads.

In order to support right chamber sensing and pacing, the lead connector 210 further comprises a right ventricular tip terminal 213 and a right ventricular ring terminal 214, which are adapted for connection to a right ventricular tip electrode and a right ventricular ring electrode of a right ventricular lead implantable in the right ventricle.

In alternative embodiments, the lead connector 210 is connectable to a left ventricular lead instead of a right ventricular lead or connectable to both a left ventricular lead and a right ventricular lead. A left ventricular lead is typically implanted in the coronary venous system for safety reasons although implantation inside the left ventricle has been proposed in the art. In the following, "left ventricular lead" is used to describe a cardiac lead designed to provide sensing and pacing functions to the left ventricle regardless of its particular implantation site, i.e. inside the left ventricle or in the coronary venous system. The left ventricular lead preferably also comprises a tip electrode and a ring electrode, which are electrically connectable to respective terminals 215, 216 of the lead connector 210.

Fig. 3 merely illustrates a typical example of cardiac lead configuration that can be used in an IMD 200. The teachings of the embodiments are not dependent on a particular lead configuration. In clear contrast, the embodiments can actually be applied to IMDs that do not have any connectable leads at all. In clear contrast, the important characteristic is that the IMD 200 comprises a communication circuitry capable of conducting wireless, RF-based communication with the communication device.

The IMD 200 as illustrated in Fig. 3 comprises an atrial pulse generator 230 and a ventricular pulse generator 232 that generate pacing pulses for delivery by the atrial lead(s) and the ventricular lead(s) preferably through an electrode configuration switch 220.

It is understood that in order to provide stimulation therapy in different heart chambers, the atrial and ventricular pulse generators 230, 232 may include dedicated, independent pulse generators, multiplexed pulse generators, or shared pulse generators. The pulse generators 230, 232 are controlled by a controller 240 via appropriate control signals, respectively, to trigger or inhibit the stimulating pulses.

The IMD 200 also comprises a controller 240, preferably in the form of a programmable microcontroller 240 that controls the operation of the IMD 200. The controller 240 typically includes a microprocessor, or equivalent control circuitry, designed specifically for controlling the delivery of pacing therapy, and may further include RAM or ROM memory, logic and timing circuitry, state machine circuitry, and I/O circuitry. Typically, the controller 240 is configured to process or monitor input signal as controlled by a program code stored in a designated memory block. The type of controller 240 is not critical to the described implementations. In clear contrast, any suitable controller may be used that carries out the functions described herein. The use of microprocessor-based control circuits for performing timing and data analysis functions are well known in the art.

The controller 240 further controls the timing of the stimulating pulses, such as pacing rate, atrioventricular interval (AVI), atrial escape interval (AEI) etc. as well as to keep track of the timing of refractory periods, blanking periods, noise detection windows, evoked response windows, alert intervals, marker channel timing, etc.

A preferred electronic configuration switch 220 includes a plurality of switches for connecting the desired terminals 211-216 to the appropriate I/O circuits, thereby providing complete electrode programmability. Accordingly, the electronic configuration switch 220, in response to a control signal from the controller 240, determines the polarity of the stimulating pulses (e.g., unipolar, bipolar, combipolar, etc.) by selectively closing the appropriate combination of switches (not shown) as is known in the art.

An atrial sensing circuit or detector 231 and a ventricular sensing circuit or detector 233 are also selectively coupled to the atrial lead(s) and the ventricular lead(s) through the switch 220 for detecting the presence of cardiac activity in the heart chambers. Accordingly, the atrial and ventricular sensing circuits 231, 233 may include dedicated sense amplifiers, multiplexed amplifiers, or shared amplifiers. The switch 220 determines the "sensing polarity" of the cardiac signal by selectively closing the appropriate switches, as is also known in the art. In this way, the clinician may program the sensing polarity independent of the stimulation polarity. The sensing circuits are optionally capable of obtaining information indicative of tissue capture as is further mentioned herein.

Each sensing circuit 231, 233 preferably employs one or more low power, precision amplifiers with programmable gain and/or automatic gain control, band-pass filtering, and a threshold detection circuit, as known in the art, to selectively sense the cardiac signal of interest.

The outputs of the atrial and ventricular sensing circuits 231, 233 are connected to the controller 240, which, in turn, is able to trigger or inhibit the atrial and ventricular pulse generators 230, 232, respectively, in a demand fashion in response to the absence or presence of cardiac activity in the appropriate chambers of the heart.

Furthermore, the controller 240 is also capable of analyzing information output from the sensing circuits 231, 233 and/or a data acquisition system 250 to determine or detect whether and to what degree tissue capture has occurred and to program a pulse, or pulse sequence, in response to such determinations. The sensing circuits 231, 233, in turn, receive control signals over signal lines from the controller 240 for purposes of controlling the gain, threshold, polarization charge removal circuitry, and the timing of any blocking circuitry coupled to the inputs of the sensing circuits 231, 233 as is known in the art.

Cardiac signals are also applied to inputs of an optional analog-to-digital (A/D) data acquisition system 250. The data acquisition system 250 is configured to acquire intracardiac electrogram signals, convert the raw analog data into a digital signal, and store the digital signals for later processing and/or transmission to the programmer by the transceiver 270. The data acquisition system 250 is coupled to the atrial lead and/or the ventricular lead through the switch 220 to sample cardiac signals across any pair of desired electrodes.

The controller 240 is further coupled to a memory 260 by a suitable data/address bus, wherein the programmable operating parameters used by the controller 240 are stored and modified, as required, in order to customize the operation of the IMD 200 to suit the needs of a particular patient. Such operating parameters define, for example, pacing pulse amplitude, pulse duration, electrode polarity, rate, sensitivity, automatic features, and time interval between pacing pulse of an applied pacing pulse sequence. The memory 260 could be the same as the memory housing the identifier codes assignable to the IMD 200 or a separate memory.

Advantageously, the operating parameters of the IMD 200 may be non-invasively programmed into the memory 260 through the transceiver 270 in communication via a communication link with the previously described communication device of the programmer.

The IMD 200 preferably also comprises an event detector 246, which may form part of the controller 240 as illustrated in the figure. The event detector 246 is configured to detect at least one pre-defined event, such as emergency event, and generate a detection signal based on the event detection. The code processor 244 is then responsive to this detection signal and is configured to switch from the first identifier code to the second identifier code as the identifier code currently assigned to the IMD 200 based on the detection signal. Detection of one of typically different pre-defined events causes the IMD 200 to change from the first or default identifier code to the second or emergency identifier code. Through this code exchange, the wakeup receiver 272 will respond to the second wakeup messages instead of the first wakeup messages. Since these second wakeup messages are preferably transmitted by the communication device more frequently than the first wakeup messages, the likelihood that a next correctly received wakeup message comprises the second identifier code is much larger as compared to a wakeup message with the first identifier code. As a consequence, it is expected that the wakeup receiver 272 shortly will capture such a second wakeup message to thereby trigger the wakeup controller 242 to activate the transceiver 270 and enable a communication session with the communication device. The IMD 200 can then transmit data packets relating to the pre-defined event detected by the event detector 246 to thereby inform the data processing unit connected to the communication device of the detected event.

The pre-defined event detectable by the event detector 246 is preferably an emergency event in terms of that it is important to send information of the detected event to the data processing unit to thereby inform the patient or a physician of the event. Examples of such events include events relating to the operation of the IMD 200 and in particular malfunction of a unit of the IMD 200. Non-limiting examples include low power of the battery 280, incorrect operation or malfunction of an IMD unit. Other example events include pre-defined medical conditions of the patient as detected by the IMD 200. These medical conditions include critical heart conditions detected by the IMD 200, including various arrhythmias, such as tachyarrhythmia and fibrillation conditions, ischemic conditions, etc.

The event detector 246 receives input from units of the IMD 200, where the input reflects physiological parameters monitored or calculated from physiological sensing in the patient. For instance, the data acquisition system 250 generate intracardiac electrogram signals of the heart from sensed electrical signals captured by electrodes of at least one cardiac lead connectable to the lead connector 210. The event detector 246 is then configured to process the intracardiac electrogram signal to detect a pre-defined heart condition of the subject based on the electrogram signal. Intracardiac electrogram signals can in particular be utilized by the event detector 246 for detecting any arrhythmia events according to techniques well-known in the art.

The controller 240 may also comprise an impedance processor (not illustrated) configured to generate an impedance signal, such as cardiogenic impedance signal, based on a voltage signal applied over a portion of the heart by two electrodes connectable to the lead connector 210 and a resulting current signal measured over a portion of the heart by two connectable electrodes. Cardiogenic impedance signals can advantageously be utilized to monitor volume changes and blood volume changes of heart chambers. The impedance signal from the impedance processor can then be used by the event detector 246 in order to detect a pre-defined medical condition.

The IMD 200 may instead or in addition comprise a sensor 290 configured to sense a physiological parameter of the patient and generate a sensor signal representative of the physiological parameter. Non-limiting example of implantable sensor that can be housed within the IMD 200 or provided connected to the IMD 200, such as provided on a lead connectable to the lead connector 210, oxygen sensors, pressure sensors, motion sensors, etc. The event detector 246 receives the sensor signal from the sensor 290 and processes it in order to detect any pre-defined event, such as an ischemic event detectable from a sensor signal representing oxygen level at a part of the heart muscle.

Information of battery status and operation state of IMD units, such as unit malfunction, is preferably provided to the event detector 246 by the controller 240.

The units of the IMD 200 can be implemented in hardware, software or a combination thereof. In particular, most of the units of the IMD 200 are typically implemented in hardware, but with units 244, 246 of the controller 240 implemented as computer program code elements or software code portions stored in the memory 260 and executed by the controller 240.

The identifier codes of the IMD are typically in the form of a sequence comprising *N* bits, such as a 24-bit sequence. An effective way of enabling multiple identifier codes for an IMD is then to have a device-specific prefix of *N*-1 bits followed by 0_{bin} or 1_{bin}, for the first or second identifier code. Thus, the first *N*-1 bits of the sequence are specific and preferably unique for the particular IMD. The last bit is then employed to discriminate between the first identifier code and the second identifier code. This concept can easily be extended to the case with more than two different identifier codes per IMD by having a device-specific prefix of *N-k* bits followed by k bits that are used to discriminate between the different *2^{k}* possible identifier codes for the IMD.

It is of course possible to use two fundamentally different and unrelated identifier codes as the first and second identifier codes, while the above described embodiments generally leads to more effective implementation of the code recognizing part of the wakeup receiver.

In certain cases wakeup messages could comprise both a manufacturer or company identifier and a transceiver identifier as identifier code. In such a case, usage of different identifier codes could be possible by having the same transceiver identifier in both types of wakeup messages but utilizing different manufacturer identifiers in the two message types.

It is also possible that the second or emergency identifier code is shared between multiple IMDs. In such a case, each IMD has a dedicated, preferably unique first identifier code, whereas two IMDs can use the same second identifier code. A non-limiting example is then to have a 24 bit sequence as identifier code with the second identifier code in the form of a sequence of 24 1_{bin}.

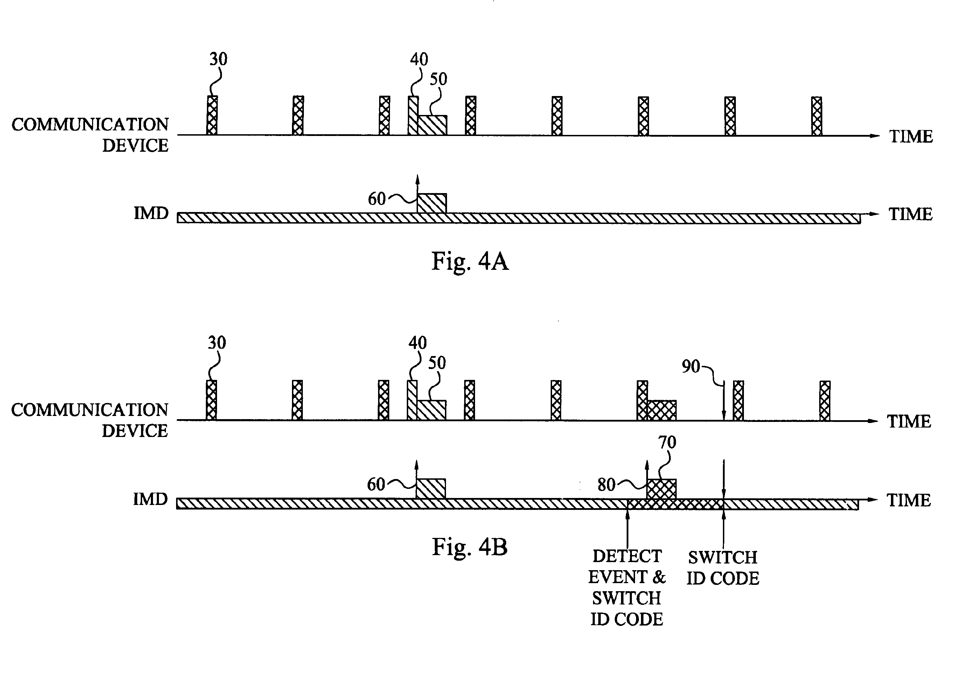

Figs. 4A to 4C schematically illustrate how the concept of using multiple identifier codes can be applied according to various embodiments. In Fig. 4A, the communication device periodically transmits first wakeup messages 40 and second wakeup messages 30. The second wakeup messages 30 are further transmitted more frequently as compared to the first wakeup messages 40. The IMD has the first identifier code as its currently assigned identifier code. As a consequence it does not respond to the second wakeup messages 30. However, the first wakeup message 40 is received by the IMD and the first identifier code therein is identified to be identical to the currently assigned identifier code. The IMD therefore activates its communication circuitry and transmits a message 60 to the communication device to inform the device that it can now initiate a communication session. The communication session involves transmission of data packets 50 between the communication device and the IMD. Once the communication session has ended, the communication circuitry of the IMD is switched to the inactive state. The following second wakeup messages 30 do not trigger any activation of the communication circuitry since the second identifier code in these messages 30 is different from the currently assigned identifier code of the IMD.

Fig. 4B illustrates the same initial procedure as in Fig. 4A. However, at a later time instance the IMD detects a pre-defined IMD operation or medical event that indicates a need for establishing a communication session. The IMD therefore switches from the first identifier code to the second identifier code to thereby be able to respond to the more frequent second wakeup messages 30. Such a second wakeup message 30 is shortly received by the IMD and triggers activation of the communication circuitry. The IMD could, for instance, respond with an activation confirmation data packet 80 and a communication session can be established. The IMD transmits data packets 70 containing information of the detected event to the communication device for further transport to the data processing unit.

After the end of the communication session, the communication device preferably generates and transmits a code setting command 90 to the IMD. The code setting command 90 triggers the IMD to switch from the second identifier code back to the first identifier code as the identifier code currently assigned to the IMD. The IMD will therefore not respond to the following second wakeup messages 30 until any further pre-defined event is detected, which trigger a switch of identifier code.

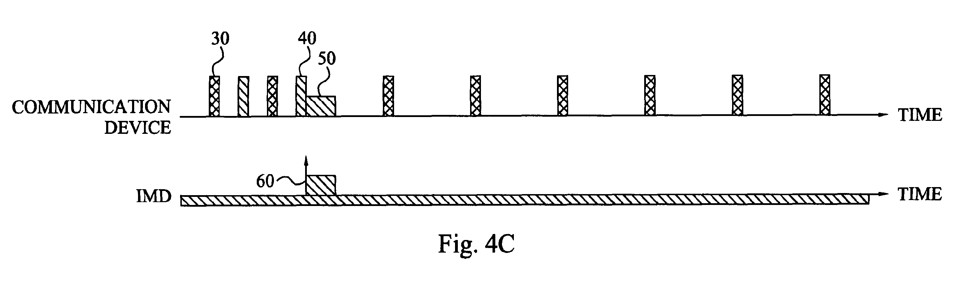

Fig. 4C illustrates the concept of the communication mode, in which the communication device wants to establish a communication session with the IMD by alternating the transmission of the first and second wakeup messages 30, 40. This concept is in particular advantageously if the wakeup receiver of the IMD is not continuously active but only active during sniffing intervals. For instance, the wakeup transceiver can be configured to be active and be able to receive messages during a sniffing interval, such as a 200 µs interval, and then enters a low power state until a next sniffing interval. The low power state is preferably significant longer than the sniffing interval, such up to about 3.5 s. By alternating the transmission of first and second wakeup messages 30, 40 the chances that a correct wakeup message with identifier code identical to the currently assigned identifier code at the IMD is received by the wakeup receiver during a sniffing interval increases. In the figure, the second first wakeup message 40 coincides with a sniffing interval and therefore triggers activation of the communication circuitry since it contains the correct identifier code.

In a particular embodiment, the communication device first transmits one of the first and second wakeup messages 30, 40 during a time period that is at least equal to the low power state plus the sniffing period. Thereafter it transmits the other of the first and second wakeup messages 30, 40 during a time period that is at least equal to the lower power state plus the sniffing period. In such a case, a communication link and session is guaranteed to be established if the IMD is within communication distance from the communication device.

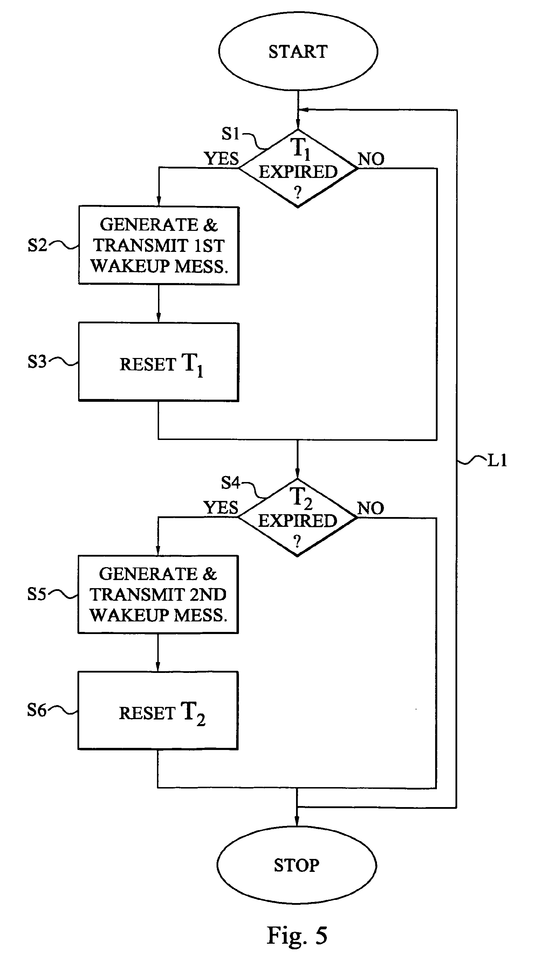

Fig. 5 is a flow diagram illustrating an embodiment of enabling wakeup of an IMD to conduct wireless RF-based communication with the IMD. The method starts in step S1 where the value of a timer T₁ associated with first wakeup messages is investigated. If the first timer T₁ has expired a new first wakeup message comprising the first identifier code is generated and transmitted in step S2. The timer T₁ is then reset in step S3. The loop of steps S1-S3 provides a periodic transmission of first wakeup messages where the periodicity is defined by the timer T₁. A corresponding loop of steps S4-S6 is employed for providing periodicity of second wakeup messages. Step S4 involves investigating whether a second timer T₂ has expired. If it has expired the method continues to step S5, where a second wakeup message comprising the second identifier code is generated and transmitted. The following step S6 resets the second timer T₂. In a preferred embodiment, the method returns to step S1 from step S4 or step S6 to thereby anew investigate the value of the first timer. The operation as illustrated in Fig. 5 corresponds to the previously mentioned default mode with separate periodicity of the two wakeup messages if T₁≠T₂. By setting T₁=T₂ the communication mode with alternate transmission of the first and second wakeup messages is achieved.

It is anticipated by the embodiments that the two loops may interchange order, i.e. steps S4-S6 are conducted first followed by steps S1-S3. The two loops can also be run in parallel.

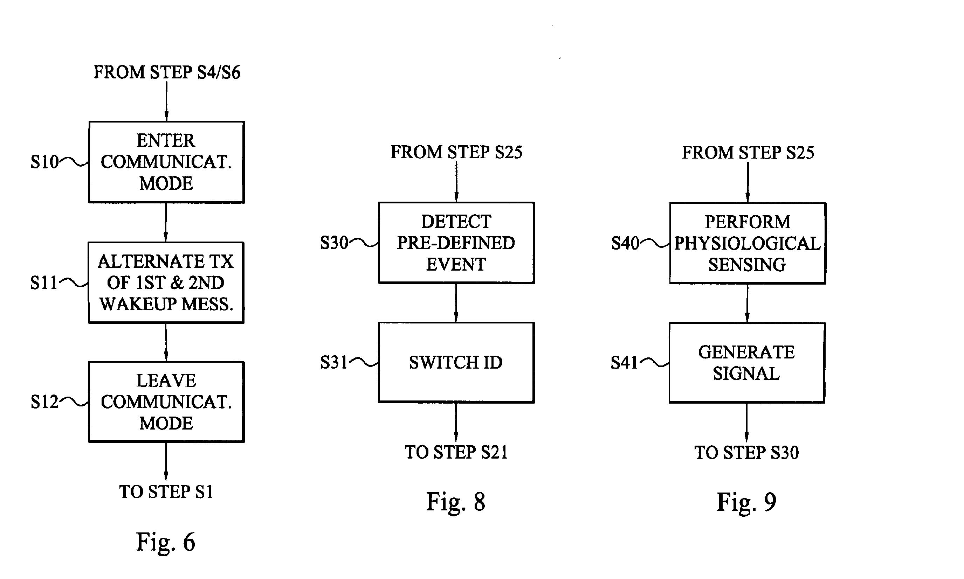

Fig. 6 illustrates additional steps referring to the communication mode. The method then continues from step S4 or S6 in Fig. 5. A next step S10 enters the communication mode, in which the communication device needs to establish a communication session with the IMD as soon as possible. Step S11 involves alternating transmission of the first and second wakeup messages, such as by setting the timers T₁=T₂. Thus, the default periodicities of the first and second wakeup messages are temporarily ignored. Once a communication session has been established and then ended the communication mode is left in step S12 and the method continues back to step S1 of Fig. 5.

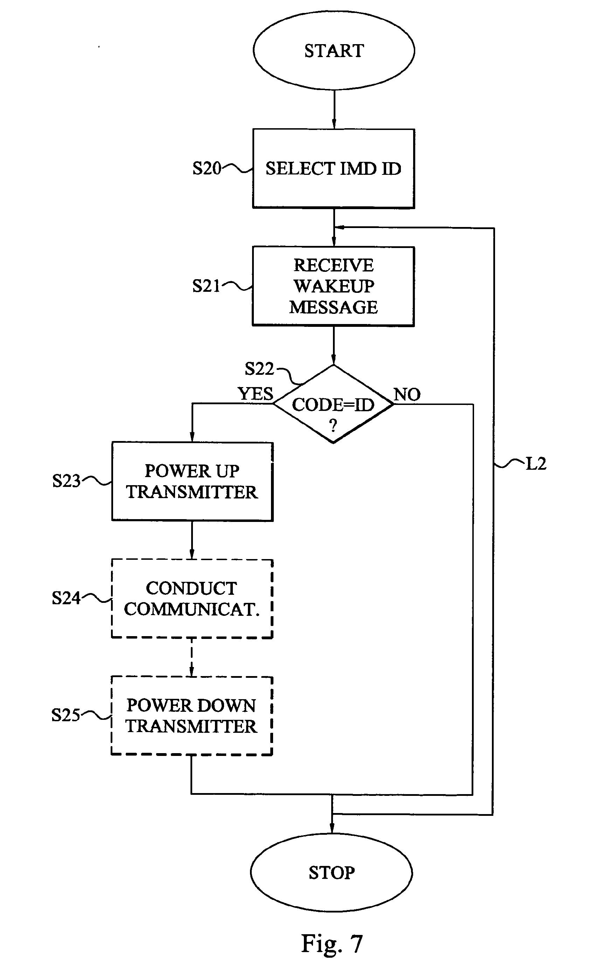

Fig. 7 is a flow diagram of method of waking up an IMD. The method starts in step S20, where the IMD selects one of multiple identifier codes as its currently assigned identifier code. In a next step S21, the IMD receives a wakeup message comprising an identifier code. The identifier code of the wakeup message is compared in step S22 to the identifier code selected in step S20. If the codes are not identical the method returns to step S21 at the time occasion of reception of a next wakeup message. If the two identifier codes, though, are identical the method continues to step S23, where the communication circuitry of the IMD, or at least the transmitter, is powered up from an inactive state to an active state. A next step S24 involves conducting communication with the communication device and after the session, the communication circuitry, i.e. at least transmitter of the IMD, is powered down to the inactive state in step S25. The method then returns to step S21, which is schematically illustrated by the line L2.

Fig. 8 illustrates additional steps of the method in Fig. 7. The method continues, for instance, from step S25 of Fig. 7. A next step S30 detects a pre-defined event triggering a need to switch identifier code in step S31, preferably from the first or default identifier code to the second or emergency identifier code. The method then returns to, for instance, step S21 of Fig. 7.

Fig. 9 illustrates additional steps of how a pre-defined event can be detected. The method continues, for instance, from step S25 of Fig. 7. In a next step S40 physiological sensing is conducted to monitor at least one physiological parameter. The sensing can be performed continuously, periodically or at defined sensing time occasions. A next step S41 generates a sensing signal representative of the physiological parameter. The method continues to step S30 of Fig. 8, where the pre-defined event is a pre-defined medical event detected based on the sensing signal.

The embodiments described above are to be understood as a few illustrative examples of the present invention. It will be understood by those skilled in the art that various modifications, combinations and changes may be made to the embodiments without departing from the scope of the present invention. In particular, different part solutions in the different embodiments can be combined in other configurations, where technically possible. The scope of the present invention is, however, defined by the appended claims.

## Claims

1. A communication device (100) configured to conduct wireless radio frequency (RF) based communication with an implantable medical device (200), comprising:
a transmitter (160) connected to an RF antenna (170);
a receiver (110) connected to an RF antenna (120);
a wakeup processor (130) connected to said transmitter (160) and configured to generate wakeup messages (30, 40), **characterized in that** said wakeup processor (130) is configured to generate first wakeup messages (40) comprising a first identifier code associated with said implantable medical device (200) and second wakeup messages (30) comprising a second, different identifier code associated with said implantable medical device (200), and said transmitter (160) is configured to transmit said first wakeup messages (40) and transmit said second wakeup messages (30).

2. The device according to claim 1, **characterized in that** said transmitter (160) is configured to transmit said first wakeup messages (40) with a first periodicity and transmit said second wakeup messages (30) with a second, different periodicity.

3. The device according to claim 1, **characterized in that** said wakeup processor (130) is configured to enter a communication mode when said communication device (100) wants to communicate data to said implantable medical device (200), wherein said transmitter (160) is configured, in said communication mode, to alternate between transmitting a first wakeup message (40) and a second wakeup message (30).

4. The device according to claim 3, **characterized in that** said wakeup processor (130) is configured to leave said communication mode following an end of a communication session involving transmission of said data to said implantable medical device (200).

5. The device according to any of the claims 1 to 4, **characterized in that** said transmitter (160) is a dedicated wakeup transmitter (160), wherein said communication device (100) comprises a transmitter (110) connected to an RF antenna (120) and configured to transmit data packets to said implantable medical device (200) during a communication session.

6. The device according to claim 5, **characterized in that** said transmitter (110) is configured to transmit a code setting command to said implantable medical device (200) following an end of a communication session, said code setting command triggering selection, at said implantable medical device (200), of said first identifier code as an identifier code currently assigned to said implantable medical device (200).

7. An implantable medical device (200) comprising:
a transmitter (270) connected to a radio frequency (RF) antenna (275);
a receiver (272) connected to an RF antenna (275);
a wakeup controller (242) connected to said transmitter (270) and configured to switch said transmitter (270) from an inactive state to an active state based on reception, by said receiver (272), of a wakeup message (30, 40) comprising an identifier code currently assigned to said implantable medical device (200), **characterized by**:
a memory (260) configured to store a first identifier code and a second, different identifier code; and
a code processor (244) connected to said memory (260) and configured to select one of said first identifier code and said second, different identifier code stored in said memory (260) as said identifier code currently assigned to said implantable medical device (200).

8. The device according to claim 7, **characterized in that** said receiver (272) is a dedicated wakeup receiver (272) at least partly active during said inactive state, said implantable medical device (200) comprises a receiver (270) connected to an RF antenna (275) and configured to receive data packets, wherein said wakeup controller (242) is connected to said transmitter (270) and said receiver (270) and is configured to switch said transmitter (270) and said receiver (270) from said inactive state to said active state based on reception, by said wakeup receiver (272), of said wakeup message (30, 40) comprising said identifier code currently assigned to said implantable medical device (200).

9. The device according to claim 7 or 8, **characterized in that** said code processor (244) is configured to select said first identifier code as a default identifier code of said implantable medical device (200).

10. The device according to claim 9, **characterized in that** said code processor (244) is configured to select said first identifier code as said identifier code currently assigned to said implantable medical device (200) based on a code setting command received from a communication device (100).

11. The device according to claim 9 or 10, **characterized by** an event detector (246) configured to detect a pre-defined event and generate a detection signal upon detection of said pre-defined event, wherein said code processor (244) is responsive to said detection signal and is configured to switch from said first identifier code to said second, different identifier code as said identifier code currently assigned to said implantable medical device (200) based on said detection signal.

12. The device according to claim 11, **characterized in that** said event detector (246) is configured to detect a pre-defined event selected from the group consisting of i) a malfunction of a unit of said implantable medical device (200) and ii) a pre-defined medical condition.

13. The device according to claim 12, **characterized by**:
a lead connector (210) connectable to at least one cardiac lead (20) having at least one electrode (22) configured to sense electrical signals from a heart (15) of a subject (10); and
a data acquisition system (250) connected to said lead connector (210) and configured to acquire intracardiac electrogram signals of said heart (15) from said sensed electrical signals, wherein said event detector (246) is configured to detect a pre-defined heart condition of said subject (10) based on said intracardiac electrogram signal.

14. The device according to claim 12, **characterized by** a sensor (290) configured to sense a physiological parameter of a subject (10) and generate a sensor signal representative of said physiological parameter, wherein said event detector (246) is configured to detect said pre-defined medical condition of said subject (10) based on said sensor signal.

15. The device according to any of the claims 1 to 14, **characterized in that** said first identifier code is a *N*-bit sequence comprising a device-specific prefix of *N*-1 bits followed by one of 0_{bin} and 1_{bin} and said second, different identifier code is a *N*-bit sequence comprising said device-specific prefix of *N*-1 bits followed by the other of 0_{bin} and 1_{bin}.

16. A method of enabling wakeup of an implantable medical device (200) to conduct wireless radio frequency (RF) based communication with said implantable medical device (200), said method comprising:
generating first wakeup messages (40) comprising a first identifier code associated with said implantable medical device (200); and
transmitting said first wakeup messages (40), **characterized by**:
generating second wakeup messages (30) comprising a second, different identifier code associated with said implantable medical device (200); and
transmitting said second wakeup messages (30).

17. The method according to claim 16, **characterized in that** said transmitting said first wakeup messages (40) comprises transmitting said first wakeup messages (40) with a first periodicity, and transmitting said second wakeup messages (30) comprises transmitting said second wakeup messages (30) with a second, different periodicity.

18. The method according to claim 16, **characterized by**:
entering a communication mode based on a need of communicating data to said implantable medical device (200), and
alternating transmitting, in said communication mode, a first wakeup message (40) and a second wakeup message (30).

19. The method according to claim 18, **characterized by** leaving said communication mode following an end of a communication session involving transmission of said data to said implantable medical device (200).

20. The method according to any of the claims 16 to 19, **characterized by** transmitting a code setting command to said implantable medical device (200), said code setting command triggering selection, at said implantable medical device (200), of said first identifier code as an identifier code currently assigned to said implantable medical device (200).

21. A method of waking up an implantable medical device (200) comprising:
receiving a wakeup message (30, 40) comprising an identifier code;
comparing said identifier code to an identifier code currently assigned to said implantable medical device (200); and
switching a transmitter (270) of said implantable medical device (200) from an inactive state to an active state if said identifier code comprised in said wakeup message (30, 40) is equal to said identifier code currently assigned to said implantable medical device (200), **characterized by** selecting one of a first identifier code and a second, different identifier code as said identifier code currently assigned to said implantable medical device (200).

22. The method according to claim 21, **characterized in that** said receiving comprises a wakeup receiver (272) of said implantable medical device (200) receiving said wakeup message (30, 40) and said switching comprises switching said transmitter (270) and a receiver (270) of said implantable medical device (200) from said inactive state to said active state if said identifier code is equal to said identifier code currently assigned to said implantable medical device (200).

23. The method according to claim 21 or 22, **characterized by** selecting said first identifier code as a default identifier code of said implantable medical device (200).

24. The method according to claim 23, **characterized by**:
receiving a code setting command following an end of a communication session; and
selecting said first identifier code as said identifier code currently assigned to said implantable medical device (200) based on said code setting command.

25. The method according to claim 23 or 24, **characterized by**:
detecting a pre-defined event; and
switching from said first identifier code to said second, different identifier code as said identifier code currently assigned to said implantable medical device (200) based on detection of said pre-defined event.

## Patentansprüche

1. Kommunikationsvorrichtung (100), welche dazu ausgelegt ist, eine auf drahtloser Hochfrequenz- (RF-) basierte Kommunikation mit einer implantierbaren medizinischen Vorrichtung (200) durchzuführen, umfassend:
einen Sender (160), der mit einer RF-Antenne (170) verbunden ist;
einen Empfänger (110), der mit einer RF-Antenne (120) verbunden ist;
einen Aufweck-Prozessor (130), der mit dem Sender (160) verbunden ist und dazu ausgelegt ist, Aufweck-Nachrichten (30, 40) zu erzeugen, **dadurch gekennzeichnet, dass** der Aufweck-Prozessor (130) dazu ausgelegt ist, erste Aufweck-Nachrichten (40), welche einen ersten Identifizierercode umfassen, der der implantierbaren medizinischen Vorrichtung (200) zugeordnet ist, und zweite Aufweck-Nachrichten (30) zu erzeugen, welche einen zweiten, unterschiedlichen Identifizierercode umfassen, der der implantierbaren medizinischen Vorrichtung (200) zugeordnet ist, und dass der Sender (160) dazu ausgelegt ist, die ersten Aufweck-Nachrichten (40) zu senden und die zweiten Aufweck-Nachrichten (30) zu senden.

2. Vorrichtung nach Anspruch 1, **dadurch gekennzeichnet, dass** der Sender (160) dazu ausgelegt ist, die ersten Aufweck-Nachrichten (40) mit einer ersten Periodizität zu senden und die zweiten Aufweck-Nachrichten (30) mit einer zweiten, unterschiedlichen Periodizität zu senden.

3. Vorrichtung nach Anspruch 1, **dadurch gekennzeichnet, dass** der Aufweck-Prozessor (130) dazu ausgelegt ist, in einen Kommunikationsmodus einzutreten, wenn die Kommunikationsvorrichtung (100) Daten zu der implantierbaren medizinischen Vorrichtung (200) zu kommunizieren wünscht, wobei der Sender (160) dazu ausgelegt ist, in dem Kommunikationsmodus zwischen einem Senden einer ersten Aufweck-Nachricht (40) und einer zweiten Aufweck-Nachricht (30) zu wechseln.

4. Vorrichtung nach Anspruch 3, **dadurch gekennzeichnet, dass** der Aufweck-Prozessor (130) dazu ausgelegt ist, den Kommunikationsmodus folgend auf ein Ende einer Kommunikationssitzung zu verlassen, welche eine Übertragung der Daten zu der implantierbaren medizinischen Vorrichtung (200) mit sich bringt.

5. Vorrichtung nach einem der Ansprüche 1 bis 4, **dadurch gekennzeichnet, dass** der Sender (160) ein zugewiesener Aufweck-Sender (160) ist, wobei die Kommunikationsvorrichtung (100) einen Sender (110) umfasst, der mit einer RF-Antenne (120) verbunden ist und dazu ausgelegt ist, Datenpakete zu der implantierbaren medizinischen Vorrichtung (200) während einer Kommunikationssitzung zu senden.

6. Vorrichtung nach Anspruch 5, **dadurch gekennzeichnet, dass** der Sender (110) dazu ausgelegt ist, einen Code-Einstellbefehl zu der implantierbaren medizinischen Vorrichtung (200) folgend auf ein Ende einer Kommunikationssitzung zu senden, wobei der Code-Einstellbefehl an der implantierbaren medizinischen Vorrichtung (200) eine Auswahl des ersten Identifizierercodes als einen Identifizierercode auslöst, der gegenwärtig der implantierbaren medizinischen Vorrichtung (200) zugeordnet ist.

7. Implantierbare medizinische Vorrichtung (200), umfassend:
einen Sender (270), der mit einer Hochfrequenz- (RF-) Antenne (275) verbunden ist;
einen Empfänger (272), der mit einer RF-Antenne (275) verbunden ist;
einen Aufweck-Controller (242), der mit dem Sender (270) verbunden ist und dazu ausgelegt ist, den Sender (270) von einem inaktiven Zustand in einen aktiven Zustand aufgrund eines Empfangs, durch den Empfänger (272), einer Aufweck-Nachricht (30, 40) zu schalten, welche einen Identifizierercode umfasst, der gegenwärtig der implantierbaren medizinischen Vorrichtung (200) zugeordnet ist, **gekennzeichnet durch**:
einen Speicher (260), welcher dazu ausgelegt ist, einen ersten Identifizierercode und einen zweiten, unterschiedlichen Identifizierercode zu speichern; und
einen Code-Prozessor (244), der mit dem Speicher (260) verbunden ist und dazu ausgelegt ist, einen von dem ersten Identifizierercode und dem zweiten, unterschiedlichen Identifizierercode, welche in dem Speicher (260) gespeichert sind, als den Identifizierercode auszuwählen, der der implantierbaren medizinischen Vorrichtung (200) zugeordnet ist.

8. Vorrichtung nach Anspruch 7, **dadurch gekennzeichnet, dass** der Empfänger (272) ein zugewiesener Aufweck-Empfänger (272) ist, der während des inaktiven Zustands zumindest teilweise aktiv ist, wobei die implantierbare medizinische Vorrichtung (200) einen Empfänger (270) umfasst, der mit einer RF-Antenne (275) verbunden ist und dazu ausgelegt ist, Datenpakete zu empfangen, wobei der Aufweck-Controller (242) mit dem Sender (270) und dem Empfänger (270) verbunden ist und dazu ausgelegt ist, den Sender (270) und den Empfänger (270) von dem inaktiven Zustand in den aktiven Zustand auf Grundlage eines Empfangs, durch den Aufweck-Empfänger (272), der Aufweck-Nachricht (30, 40) zu schalten, welche den Identifizierercode umfasst, der gegenwärtig der implantierbaren medizinischen Vorrichtung (200) zugeordnet ist.

9. Vorrichtung nach Anspruch 7 oder 8, **dadurch gekennzeichnet, dass** der Code-Prozessor (244) dazu ausgelegt ist, den ersten Identifizierercode als einen Vorgabe-Identifizierercode der implantierbaren medizinischen Vorrichtung (200) auszuwählen.

10. Vorrichtung nach Anspruch 9, **dadurch gekennzeichnet, dass** der Code-Prozessor (244) dazu ausgelegt ist, den ersten Identifizierercode als den Identifizierercode auszuwählen, der gegenwärtig der implantierbaren medizinischen Vorrichtung (200) zugeordnet ist, auf Grundlage eines Code-Einstellbefehls, der von einer Kommunikationsvorrichtung (100) empfangen wird.

11. Vorrichtung nach Anspruch 9 oder 10, **gekennzeichnet durch** einen Ereignisdetektor (246), der dazu ausgelegt ist, ein vordefiniertes Ereignis zu erfassen und ein Erfassungssignal auf eine Erfassung des vordefinierten Ereignisses hin zu erzeugen, wobei der Code-Prozessor (244) ansprechend auf das Erfassungssignal ist und dazu ausgelegt ist, auf Grundlage des Erfassungssignals von dem ersten Identifizierercode auf den zweiten, unterschiedlichen Identifizierercode als den Identifizierercode zu schalten, der gegenwärtig der implantierbaren medizinischen Vorrichtung (200) zugeordnet ist.

12. Vorrichtung nach Anspruch 11, **dadurch gekennzeichnet, dass** der Ereignisdetektor (246) dazu ausgelegt ist, ein vordefiniertes Ereignis zu erfassen, das gewählt ist aus der Gruppe, welche besteht aus i) einer Fehlfunktion einer Einheit der implantierbaren medizinischen Vorrichtung (200) und ii) einem vordefinierten medizinischen Zustand.

13. Vorrichtung nach Anspruch 12, **gekennzeichnet durch**:
einen Leitungsverbinder (210), der mit einer kardialen Leitung (20) verbindbar ist, welche zumindest eine Elektrode (22) aufweist, die dazu ausgelegt ist, elektrische Signale von einem Herzen (15) eines Probanden (10) zu erfassen; und
ein Datenerfassungssystem (250), das mit dem Leitungsverbinder (210) verbunden ist und dazu ausgelegt ist, Herz-Elektrogrammsignale des Herzens (15) aus den erfassten elektrischen Signalen zu erlangen, wobei der Ereignisdetektor (246) dazu ausgelegt ist, einen vordefinierten Herzzustand des Probanden (10) auf Grundlage des Herz-Elektrogrammsignals zu erfassen.

14. Vorrichtung nach Anspruch 12, **gekennzeichnet durch** einen Sensor (290), welcher dazu ausgelegt ist, einen physiologischen Parameter eines Probanden (10) zu erfassen und ein Sensorsignal zu erzeugen, das repräsentativ für den physiologischen Parameter ist, wobei der Ereignisdetektor (246) dazu ausgelegt ist, den vordefinierten medizinischen Zustand des Probanden (10) auf Grundlage des Sensorsignals zu erfassen.

15. Vorrichtung nach einem der Ansprüche 1 bis 14, **dadurch gekennzeichnet, dass** der erste Identifizierercode eine N-Bit-Sequenz ist, welche einen Vorrichtungs-spezifischen Präfix von N-1 Bits, gefolgt von einem von 0_{bin} und 1_{bin} umfasst, und dass der zweite, unterschiedliche Identifizierercode eine N-Bit-Sequenz ist, welche den Vorrichtungs-spezifischen Präfix von N-1 Bits gefolgt von dem jeweils anderen des 0_{bin} und 1_{bin} umfasst.

16. Verfahren zum Ermöglichen eines Aufweckens einer implantierbaren medizinischen Vorrichtung (200), um eine auf drahtloser Hochfrequenz- (RF-) basierte Kommunikation mit der implantierbaren medizinischen Vorrichtung (200) durchzuführen, wobei das Verfahren umfasst:
Erzeugen erster Aufweck-Nachrichten (40), welche einen ersten Identifizierercode umfassen, der der implantierbaren medizinischen Vorrichtung (200) zugeordnet ist; und
Senden der ersten Aufweck-Nachrichten (40), **gekennzeichnet durch**:
Erzeugen zweiter Aufweck-Nachrichten (30), welche einen zweiten, unterschiedlichen Identifizierercode umfassen, welcher der implantierbaren medizinischen Vorrichtung (200) zugeordnet ist; und
Senden der zweiten Aufweck-Nachrichten (30).

17. Verfahren nach Anspruch 16, **dadurch gekennzeichnet, dass** das Senden der ersten Aufweck-Nachrichten (40) ein Senden der ersten Aufweck-Nachrichten (40) mit einer ersten Periodizität umfasst und ein Senden der zweiten Aufweck-Nachrichten (30), ein Senden der zweiten Aufweck-Nachrichten (30) mit einer zweiten, unterschiedlichen Periodizität umfasst.

18. Verfahren nach Anspruch 16, **gekennzeichnet durch**:
Eintreten in einen Kommunikationsmodus auf Grundlage eines Erfordernisses zum Kommunizieren von Daten zu der implantierbaren medizinischen Vorrichtung (200), und
abwechselndes Senden, in dem Kommunikationsmodus, einer ersten Aufweck-Nachricht (40) und einer zweiten Aufweck-Nachricht (30).

19. Verfahren nach Anspruch 18, **gekennzeichnet durch** ein Verlassen des Kommunikationsmodus auf ein Ende einer Kommunikationssitzung folgend, welche eine Übertragung der Daten zu der implantierbaren medizinischen Vorrichtung (200) mit sich bringt.

20. Verfahren nach einem der Ansprüche 16 bis 19, **gekennzeichnet durch** ein Senden eines Code-Einstellbefehls zu der implantierbaren medizinischen Vorrichtung (200), wobei der Code-Einstellbefehl an der implantierbaren medizinischen Vorrichtung (200) eine Auswahl des ersten Identifizierercodes als einen Identifizierercode auslöst, der gegenwärtig der implantierbaren medizinischen Vorrichtung (200) zugeordnet ist.

21. Verfahren zum Aufwecken einer implantierbaren medizinischen Vorrichtung (200), umfassend:
Empfangen einer Aufweck-Nachricht (30, 40), welche einen Identifizierercode umfasst;
Vergleichen des Identifizierercodes mit einem Identifizierercode, der gegenwärtig der implantierbaren medizinischen Vorrichtung (200) zugeordnet ist; und
Schalten eines Senders (270) der implantierbaren medizinischen Vorrichtung (200) von einem inaktiven Zustand in einen aktiven Zustand, wenn der Identifizierercode, der in der Aufweck-Nachricht (30, 40) enthalten ist, gleich dem Identifizierercode ist, der gegenwärtig der implantierbaren medizinischen Vorrichtung (200) zugeordnet ist, **gekennzeichnet durch** ein Auswählen eines eines ersten Identifizierercodes und eines zweiten, unterschiedlichen Identifizierercodes als den Identifizierercode, der gegenwärtig der implantierbaren medizinischen Vorrichtung (200) zugeordnet ist.

22. Verfahren nach Anspruch 21, **dadurch gekennzeichnet, dass** das Empfangen umfasst, dass ein Aufweck-Empfänger (272) der implantierbaren medizinischen Vorrichtung (200) die Aufweck-Nachricht (30, 40) empfängt, und das Schalten ein Schalten des Senders (270) und eines Empfängers (270) der implantierbaren medizinischen Vorrichtung (200) von dem inaktiven Zustand in den aktiven Zustand umfasst, wenn der Identifizierercode gleich dem Identifizierercode ist, der gegenwärtig der implantierbaren medizinischen Vorrichtung (200) zugeordnet ist.

23. Verfahren nach Anspruch 21 oder 22, **gekennzeichnet durch** ein Auswählen des ersten Identifizierercodes als einen Vorgabe-Identifizierercode der implantierbaren medizinischen Vorrichtung (200).

24. Verfahren nach Anspruch 23, **gekennzeichnet durch**:
Empfangen eines Code-Einstellbefehls folgend auf ein Ende einer Kommunikationssitzung; und
Auswählen des ersten Identifizierercodes als den Identifizierercode, der gegenwärtig der implantierbaren medizinischen Vorrichtung (200) zugewiesen ist, auf Grundlage des Code-Einstellbefehls.

25. Verfahren nach Anspruch 23 oder 24, **gekennzeichnet durch**:
Erfassen eines vordefinierten Ereignisses; und
Schalten von dem ersten Identifizierercode in den zweiten, unterschiedlichen Identifizierercode als den Identifizierercode, der gegenwärtig der implantierbaren medizinischen Vorrichtung (200) zugeordnet ist, auf Grundlage einer Erfassung des vordefinierten Ereignisses.

## Revendications

1. Dispositif de communication (100) configuré pour réaliser une communication sans fil par radiofréquence (RF) avec un dispositif médical implantable (200), comprenant :
un émetteur (160) connecté à une antenne RF (170) ;
un récepteur (110) connecté à une antenne RF (120) ;
un processeur de réveil (130) connecté audit émetteur (160) et configuré pour générer des messages de réveil (30, 40), **caractérisé en ce que** ledit processeur de réveil (130) est configuré pour générer des premiers messages de réveil (40) comprenant un premier code d'identification associé audit dispositif médical implantable (200) et des seconds messages de réveil (30) comprenant un second code d'identification différent associé audit dispositif médical implantable (200), et ledit émetteur (160) est configuré pour émettre lesdits premiers messages de réveil (40) et lesdits seconds messages de réveil (30).

2. Dispositif selon la revendication 1, **caractérisé en ce que** ledit émetteur (160) est configuré pour émettre lesdits premiers messages de réveil (40) avec une première périodicité et émettre lesdits seconds messages de réveil (30) avec une seconde périodicité différente.

3. Dispositif selon la revendication 1, **caractérisé en ce que** ledit processeur de réveil (130) est configuré pour passer à un mode de communication lorsque ledit dispositif de communication (100) veut communiquer des données audit dispositif médical implantable (200), ledit émetteur (160) étant configuré, dans ledit mode de communication, pour alterner entre l'émission d'un premier message de réveil (40) et l'émission d'un second message de réveil (30).

4. Dispositif selon la revendication 3, **caractérisé en ce que** ledit processeur de réveil (130) est configuré pour sortir dudit mode de communication suite à la fin d'une session de communication impliquant l'émission desdites données à destination dudit dispositif médical implantable (200).

5. Dispositif selon l'une quelconque des revendications 1 à 4, **caractérisé en ce que** ledit émetteur (160) est un émetteur de réveil (160) dédié, ledit dispositif de communication (100) comprenant un émetteur (110) connecté à une antenne RF (120) et configuré pour émettre des paquets de données à destination dudit dispositif médical implantable (200) pendant une session de communication.

6. Dispositif selon la revendication 5, **caractérisé en ce que** ledit émetteur (110) est configuré pour émettre une commande d'établissement de code à destination dudit dispositif médical implantable (200) suite à la fin d'une session de communication, ladite commande d'établissement de code déclenchant la sélection, au niveau dudit dispositif médical implantable (200), dudit premier code d'identification en tant que code d'identification actuellement assigné audit dispositif médical implantable (200).

7. Dispositif médical implantable (200) comprenant :
un émetteur (270) connecté à une antenne radiofréquence (RF) (275) ;
un récepteur (272) connecté à une antenne RF (275) ;
un organe de commande de réveil (242) connecté audit émetteur (270) et configuré pour faire basculer ledit émetteur (270) d'un état inactif à un état actif en fonction de la réception, par ledit récepteur (272), d'un message de réveil (30, 40) comprenant un code d'identification actuellement assigné audit dispositif médical implantable (200), **caractérisé par** :
une mémoire (260) configurée pour stocker un premier code d'identification et un second code d'identification différent ; et
un processeur de code (244) connecté à ladite mémoire (260) et configuré pour choisir ledit premier code d'identification ou ledit second code d'identification différent stocké dans ladite mémoire (260) en tant que ledit code d'identification actuellement assigné audit dispositif médical implantable (200).

8. Dispositif selon la revendication 7, **caractérisé en ce que** ledit récepteur (272) est un récepteur de réveil (272) dédié qui est au moins partiellement actif pendant ledit état inactif, ledit dispositif médical implantable (200) comprend un récepteur (270) connecté à une antenne RF (275) et configuré pour recevoir des paquets de données, ledit organe de commande de réveil (242) étant connecté audit émetteur (270) et audit récepteur (270) et étant configuré pour faire basculer ledit émetteur (270) et ledit récepteur (270) dudit état inactif audit état actif en fonction de la réception, par ledit récepteur de réveil (272), dudit message de réveil (30, 40) comprenant ledit code d'identification actuellement assigné audit dispositif médical implantable (200).

9. Dispositif selon la revendication 7 ou 8, **caractérisé en ce que** ledit processeur de code (244) est configuré pour choisir ledit premier code d'identification en tant que code d'identification par défaut dudit dispositif médical implantable (200).

10. Dispositif selon la revendication 9, **caractérisé en ce que** ledit processeur de code (244) est configuré pour choisir ledit premier code d'identification en tant que ledit code d'identification actuellement assigné audit dispositif médical implantable (200) en fonction d'une commande d'établissement de code provenant d'un dispositif de communication (100).

11. Dispositif selon la revendication 9 ou 10, **caractérisé par** un détecteur d'événement (246) configuré pour détecter un événement prédéfini et générer un signal de détection en cas de détection dudit événement prédéfini, ledit processeur de code (244) réagissant audit signal de détection et étant configuré pour faire basculer ledit premier code d'identification en ledit second code d'identification différent en tant que ledit code d'identification actuellement assigné audit dispositif médical implantable (200) en fonction dudit signal de détection.

12. Dispositif selon la revendication 11, **caractérisé en ce que** ledit détecteur d'événement (246) est configuré pour détecter un événement prédéfini choisi dans le groupe constitué par i) un dysfonctionnement d'une unité dudit dispositif médical implantable (200) et ii) une pathologie prédéfinie.

13. Dispositif selon la revendication 12, **caractérisé par** :
un connecteur de dérivation (210) susceptible d'être raccordé à au moins une dérivation cardiaque (20) présentant au moins une électrode (22) configurée pour détecter les signaux électriques du coeur (15) d'un sujet (10) ; et
un système d'acquisition de données (250) connecté audit connecteur de dérivation (210) et configuré pour acquérir des signaux d'électrogramme intracardiaque dudit coeur (15) à partir desdits signaux électriques détectés, ledit détecteur d'événement (246) étant configuré pour détecter une pathologie cardiaque prédéfinie dudit sujet (10) en fonction dudit signal d'électrogramme intracardiaque.

14. Dispositif selon la revendication 12, **caractérisé par** un capteur (290) configuré pour capter un paramètre physiologique chez un sujet (10) et générer un signal de détection représentatif dudit paramètre physiologique, ledit détecteur d'événement (246) étant configuré pour détecter ladite pathologie prédéfinie dudit sujet (10) en fonction dudit signal de détection.

15. Dispositif selon l'une quelconque des revendications 1 à 14, **caractérisé en ce que** ledit premier code d'identification est une séquence comportant *N* bits, comprenant un préfixe spécifique au dispositif de *N*-1 bits suivi soit par 0_{bin} soit par 1_{bin} et ledit second code d'identification différent est une séquence comportant *N* bits, comprenant ledit préfixe spécifique au dispositif de *N*-1 bits inversement suivi soit par 0_{bin} soit par 1_{bin}.

16. Procédé permettant le réveil d'un dispositif médical implantable (200) afin de réaliser une communication sans fil par radiofréquence (RF) avec ledit dispositif médical implantable (200), ledit procédé comprenant :
la génération de premiers messages de réveil (40) comprenant un premier code d'identification associé audit dispositif médical implantable (200) ; et
l'émission desdits premiers messages de réveil (40), **caractérisé par** :
la génération de seconds messages de réveil (30) comprenant un second code d'identification différent associé audit dispositif médical implantable (200) ; et
l'émission desdits seconds messages de réveil (30).

17. Procédé selon la revendication 16, **caractérisé en ce que** ladite émission desdits premiers messages de réveil (40) comprend l'émission desdits premiers messages de réveil (40) avec une première périodicité, et l'émission desdits seconds messages de réveil (30) comprend l'émission desdits seconds messages de réveil (30) avec une seconde périodicité différente.

18. Procédé selon la revendication 16, **caractérisé par** :
le passage à un mode de communication en fonction du besoin de communiquer des données audit dispositif médical implantable (200), et
l'alternance de l'émission, dans ledit mode de communication, entre un premier message de réveil (40) et un second message de réveil (30).

19. Procédé selon la revendication 18, **caractérisé par** la sortie dudit mode de communication suite à la fin d'une session de communication impliquant l'émission desdites données auprès dudit dispositif médical implantable (200).

20. Procédé selon l'une quelconque des revendications 16 à 19, **caractérisé par** l'émission d'une commande d'établissement de code auprès dudit dispositif médical implantable (200), ladite commande d'établissement de code déclenchant la sélection, au niveau dudit dispositif médical implantable (200), dudit premier code d'identification en tant que code d'identification actuellement assigné audit dispositif médical implantable (200).

21. Procédé de réveil d'un dispositif médical implantable (200) comprenant :
la réception d'un message de réveil (30, 40) comprenant un code d'identification ;
la comparaison dudit code d'identification à un code d'identification actuellement assigné audit dispositif médical implantable (200) ; et
le basculement d'un émetteur (270) dudit dispositif médical implantable (200), d'un état inactif à un état actif, si ledit code d'identification compris dans ledit message de réveil (30, 40) est égal audit code d'identification actuellement assigné audit dispositif médical implantable (200), **caractérisé par** la sélection d'un premier code d'identification ou d'un second code d'identification différent en tant que ledit code d'identification actuellement assigné audit dispositif médical implantable (200).

22. Procédé selon la revendication 21, **caractérisé en ce que** ladite réception comprend le fait qu'un récepteur de réveil (272) dudit dispositif médical implantable (200) reçoit ledit message de réveil (30, 40) et ledit basculement comprend le basculement dudit émetteur (270) et d'un récepteur (270) dudit dispositif médical implantable (200), dudit état inactif audit état actif, si ledit code d'identification est égal audit code d'identification actuellement assigné audit dispositif médical implantable (200).

23. Procédé selon la revendication 21 ou 22, **caractérisé par** la sélection dudit premier code d'identification en tant que code d'identification par défaut dudit dispositif médical implantable (200).

24. Procédé selon la revendication 23, **caractérisé par** :
la réception d'une commande d'établissement de code suite à la fin d'une session de communication ; et
la sélection dudit premier code d'identification en tant que ledit code d'identification actuellement assigné audit dispositif médical implantable (200) en fonction de ladite commande d'établissement de code.

25. Procédé selon la revendication 23 ou 24, **caractérisé par** :
la détection d'un événement prédéfini ; et
le basculement, dudit premier code d'identification audit second code d'identification différent, en tant que ledit code d'identification actuellement assigné audit dispositif médical implantable (200) en fonction de la détection dudit événement prédéfini.
